# EUROPEAN PATENT SPECIFICATION

(11) **EP 1 890 987 B1**
(45) Date of publication and mention of the grant of the patent: **26.03.2014**
(21) Application number: 06758866.5
(22) Date of filing: 01.05.2006
(51) Int. Cl.: C07C 19/075, C07C 17/395, C07C 17/16, C07C 17/42, C07C 17/38

(54) **1-bromopropane having low acidity**
1-Bromopropan mit niedriger Acidität
1-Bromopropane faiblement acide

(30) Priority: 03.05.2005 US 677076 P
(43) Date of publication of application: 27.02.2008
(73) Proprietor: ALBEMARLE CORPORATION, Baton Rouge Louisiana 70801-1765 (US)
(72) Inventor: MUTTERER, Vincent, Luc, F-68128 Rosenau (FR); TRITZ, Jean-Philippe, F-68170 Rixheim (FR)
(74) Representative: UEXKÜLL & STOLBERG
(86) International application number: PCT/US2006/016669
(87) International publication number: WO 2006/119213

(56) References cited:
- WO-A-02/22530
- WO-A-2006/052241
- US-A- 5 138 110
- US-A- 6 049 014
- US-A1- 2002 151 447
- US-A1- 2005 065 386
- US-B1- 6 365 565
- PATENT ABSTRACTS OF JAPAN vol. 1997, no. 04, 30 April 1997 (1997-04-30) & JP 08 337795 A (TOSOH CORP), 24 December 1996 (1996-12-24)
- PATENT ABSTRACTS OF JAPAN vol. 014, no. 074 (C-0687), 13 February 1990 (1990-02-13) & JP 01 292095 A (DAIKIN IND LTD), 24 November 1989 (1989-11-24)
- PATENT ABSTRACTS OF JAPAN vol. 1996, no. 04, 30 April 1996 (1996-04-30) & JP 07 330640 A (DAIKIN IND LTD), 19 December 1995 (1995-12-19)

## Description

### TECHNICAL FIELD

This invention relates to 1-bromopropane products having a reduced tendency for acid formation during storage, and to processes for producing such products.

WO 02/22530 and US 2002/0151447 both describe 1-bromopropane with a lower isopropyl bromide content via distillation of the starting material (n-propanol) for forming the 1-bromopropane. US 6,365,565 discloses mixtures of 1-bromopropane and one or more other solvents, especially combinations which have azeotrope-like properties. In US 6,049,014, n-propyl bromide is co-produced during the bromination of bisphenols and trisphenols. A high yield of n-propyl bromide is obtained in US 5,138,110 via a continuous feed of HBr and n-propanol. US 2005/0065386 describes n-propyl bromide having less than 0.1 wt.% isopropyl bromide, where the n-propyl bromide is made from n-propanol and excess HBr. WO 2006/052241 discloses the use of substituted phenolic compounds to stabilize n-propyl bromide. JP 08-337795 describes 1-bromopropane stabilized with nitromethane and either butylene oxide or trimethoxymethane.

Patent abstracts of Japan Vol. 1996, no. 04, April 30 1996 and JP 07 330640 A of Daikin Ind. Ltd., December 19, 1995, disclose a method for removing 1,1-dichloroethylene and purifying 1,1-dichloro-1-fluoroethane by bringing dichloro-fluoroethane containing dichloroethylene into contact with an aqueous solution containing a metal permanganate and a metal hydroxide, oxidizing dichloroethylene and separating dichloro-fluoroethane from the oxidized product of dichloroethylene.

### BACKGROUND

1-Bromopropane (also referred to as n-propyl bromide or propyl bromide) can be used as a degreasing agent, especially for degreasing metal parts, as well as in cleaning solutions for electrical circuit board production. In such applications, the presence of certain impurities in the 1-bromopropane is undesirable because the impurities have a detrimental effect on the substrates with which the 1-bromopropane is brought into contact. When manufactured, an initial 1-bromopropane product mixture contains impurities, including one or more of 2-bromopropane (isopropyl bromide), 1,2-dibromopropane, monobromoacetone, 1-propanol, propionaldehyde, propionic acid, water, and hydrogen bromide (HBr). Some of these impurities can, over time, degrade to yield acids, such as HBr and propionic acid. Thus, trace amounts of HBr are often present in finished 1-bromopropane products; further, the acidity increases over time in a finished 1-bromopropane product Acidic species are detrimental to the performance qualities of 1-bromopropane as they can corrode or discolor the substrates to which the 1-bromopropane is applied. The art has tried to address this problem by adding acid scavenging compounds, such as 1,2-epoxides to the 1-bromopropane. Metal passivators, such as nitromethane and 1,3-dioxolane, have also been used. The problem with such additives is that there is a cost associated with their use, and they are consumed as they act to reduce acid content in the 1-bromopropane product. The consumption rate of the additive(s) can be such that, after a period of time, there is no further additive to reduce acid presence in the 1-bromopropane product. In this condition, the acid content of the 1-bromopropane can increase to deleterious levels. Thus, a better method for minimizing the acidic impurities in 1-bromopropane is needed, particularly one that does not compromise performance of the 1-bromopropane.

### SUMMARY OF THE INVENTION

This invention provides 1-bromopropane products having a reduced tendency to produce acidic species over time. In particular, the invention can provide 1-bromopropane products that meet the requirement of keeping the acidity below 10 ppm for at least 30 days at 60°C without detracting from the performance of the finished 1-bromopropane product. Surprisingly, it has been found that contacting a 1-bromopropane product mixture with permanganate, an oxidant, reduces or prevents the formation of acidity in a 1-bromopropane product produced therefrom. In particular, when a 1-bromopropane product mixture is treated with permanganate pursuant to this invention, the acidity of the 1-bromopropane product is reduced (in comparison to a 1-bromopropane product not so treated), and often the acidity of a permanganate-treated 1-bromopropane product remains below 10 ppm for at least 30 days at 60°C. As used herein, the term "1-bromopropane product" denotes a 1-bromopropane product mixture that has been treated with permanganate, had the permanganate removed therefrom, and optionally has been subjected to further purification. Similarly, the term "1-bromopropane product mixture" denotes generally 1-bromopropane containing one or more of the impurities that are formed therewith during the course of manufacture of 1-bromopropane. More specifically, the 1-bromopropane product mixtures used in the processes of this invention generally contain 1-bromopropane and one or more of the impurities that are formed therewith during the course of manufacture of 1-bromopropane; the impurities are as described above.

This invention relates in part to processes for removing acidic and/or acid-forming species from 1-bromopropane product mixtures formed from 1-propanol and HBr. The processes of the invention result in the reduction of acidic species and/or acid-forming species, including propanol, propionaldehyde, and propionic acid. For example, 1-bromopropane product mixtures formed from 1-propanol and HBr typically contain propanol, propionaldehyde, and propionic acid. Thus, practicing the process of this invention upon 1-bromopropane product mixtures made by such process is advantageous.

Pursuant to this invention, a 1-bromopropane product mixture resulting from a 1-bromopropane production process from 1-propanol and HBr is mixed with permanganate in proportions of 0.1 to 5 parts by weight permanganate per 1000 parts by weight of 1-bromopropane product mixture. After the mixing, purification steps are taken to reduce the content of the permanganate oxidation products and unreacted permanganate in the 1-bromopropane product mixture. The 1-bromopropane product is obtained from the pennanganate-contacted 1-bromopropane product mixture. If desired, conventional additives such as acid scavengers and metal passivators can be added to the 1-bromopropane products of this invention.

An embodiment of this invention is a process which comprises mixing permanganate with a 1-bromopropane product mixture to form a permanganate-containing 1-bromopropane product mixture, and recovering a 1-bromopropane product from the permanganate-containing 1-bromopropane product mixture.

In this invention, as used herein, the term "permanganate-containing 1-bromopropane product mixture" denotes a 1-bromopropane product mixture with which permanganate has been mixed, but which 1-bromopropane product mixture has not been separated from the permanganate, *i.e*., permanganate, in one form or another, is present in the 1-bromopropane product mixture. Similarly, the term "1-bromopropane product," as used herein, denotes a 1-bromopropane product mixture that has been mixed with permanganate, and from which the permanganate has been separated. Usually and preferably, the 1-bromopropane product undergoes further purification.

Thus, this invention is a process for preparing a 1-bromopropane product mixture resulting from a 1-bromopropane production process from 1-propanol and hydrogen bromide, characterized by mixing permanganate in the indicated amount with the 1-bromopropane product mixture to form a permanganate-containing 1-bromopropane product mixture, and recovering a 1-bromopropane product from the permanganate-containing 1-bromopropane product mixture.

Still another embodiment of this invention is a composition comprising permanganate and a 1-bromopropane product mixture.

These and other embodiments and features of this invention will be still further apparent from the ensuing description and appended claims.

### FURTHER DETAILED DESCRIPTION OF THE INVENTION

The term ppm means parts per million (wt/wt), unless specifically stated otherwise herein.

It is believed, though this invention is not bound by any theory of mechanism, that permanganate oxidizes at least two of the acid-forming impurities often found in 1-bromopropane products, be they crude products or refined products containing acid-reducing additives. Impurities that seem to contribute to the acidity of prior art 1-bromopropane products include 1-propanol and propionaldehyde. It is believed that the permanganate oxidizes these impurities to propionic acid, which can be reduced or removed by washing the 1-bromopropane product mixture with an aqueous solution of an inorganic base; this aqueous base wash will also remove unreacted permanganate from the 1-bromopropane product mixture. Unreacted permanganate can also be removed or reduced by washing the permanganate-containing 1-bromopropane product mixture with water.

Preferred 1-bromopropane product mixtures used in this invention have a purity of at least about 90%, and more preferably a purity of at least about 95%, and still more preferably a purity of at least about 98%, the balance in each case being one or more impurities resulting from the process by which the 1-bromopropane product mixture was prepared.

Permanganate can be mixed with a 1-bromopropane product mixture at almost any point after formation of the 1-bromopropane product mixture. While permanganate can be mixed with a crude 1-bromopropane product mixture (*i.e*., prior to any purification of the 1-bromopropane product mixture), normally and preferably the crude 1-bromopropane product mixture is washed with water and/or with an aqueous solution of at least one inorganic base prior to mixing with permanganate. More than one wash of the 1-bromopropane product mixture, or other purification steps, such as distillation, may be carried out before mixing permanganate with the 1-bromopropane product mixture.

For the mixing of permanganate with a 1-bromopropane product mixture, permanganate can be added to the 1-bromopropane product mixture, the 1-bromopropane product mixture can be added to permanganate, or permanganate and the 1-bromopropane product mixture can be co-fed to a vessel or mixing zone. Preferably, permanganate is added to a 1-bromopropane product mixture.

Basic conditions are preferred for mixing the permanganate with a 1-bromopropane product mixture. The pH is preferably in range of 8 to 10; higher pH values are acceptable but not necessary. By "basic conditions" it is meant that when water or an aqueous solution is stirred with a 1-bromopropane product mixture, and the aqueous and organic phases are allowed to separate, the pH of the aqueous phase is above 7. Basic conditions are typically achieved by performing one or more washes of a 1-bromopropane product mixture with an aqueous solution of an inorganic base, preferably an alkali metal base, more preferably an alkali metal hydroxide. Mixing of permanganate with a 1-bromopropane product mixture under acidic conditions is acceptable, but not preferred.

A 1-bromopropane product is recovered from a permanganate-containing 1-bromopropane product mixture by separating the permanganate from the 1-bromopropane product mixture. The separation can be accomplished in various ways, including distillation and washing. Washing the permanganate-containing 1-bromopropane product mixture with water or an aqueous solution of an inorganic base is a preferred way to separate the permanganate to obtain a purified 1-bromopropane product. An advantage to operation under basic conditions is that a water wash or a wash with an aqueous solution of an inorganic base of the permanganate-containing 1-bromopropane product mixture will extract propionic acid from the permanganate-containing 1-bromopropane product mixture. Without wishing to be bound by theory, permanganate is thought to oxidize propanol and propionaldehyde to propionic acid; thus, washing the permanganate-containing 1-bromopropane product mixture with water or an aqueous solution of an inorganic base will remove propionic acid so formed (in addition to removing unconsumed permanganate). Further purification steps such as distillation or one or more additional washes with water and/or an aqueous solution of an inorganic base may be performed on the 1-bromopropane product.

In the processes of this invention, drying is normally the final step in the purification of a 1-bromopropane product, after all of the other purification steps, if any, have been performed. It is generally not desirable to mix permanganate with a 1-bromopropane product mixture after the drying step, because permanganate will remain in the 1-bromopropane product.

Generally, drying of a 1-bromopropane product mixture pursuant to the processes of this invention is carried out by contacting the 1-bromopropane product with a drying agent. Suitable drying agents are well known in the art and include calcium chloride, magnesium sulfate, calcium sulfate, potassium carbonate, and the like. A preferred drying agent in the practice of this invention is calcium chloride. Preferably, drying continues until the water content of the 1-bromopropane product is less than about 100 ppm (wt/wt); more preferably, drying continues until the water content of the 1-bromopropane product is less than about 80 ppm (wt/wt).

In some preferred processes of the invention, a 1,2-epoxide is mixed with the 1-bromopropane product. The 1,2-epoxide can be mixed into a 1-bromopropane product before or after the drying step, or a portion of the 1,2-epoxide can be mixed with the 1-bromopropane product before the drying step, and another portion (the remainder) of the 1,2-epoxide can be mixed with the 1-bromopropane product after the drying step.

In the practice of this invention, permanganate, an anion, is mixed with a 1-bromopropane product mixture as one or more of its salts. Suitable permanganate salts include lithium permanganate, sodium permanganate, potassium permanganate, rubidium permanganate, cesium permanganate, calcium permanganate, magnesium permanganate, zinc permanganate, silver permanganate, lanthanum permanganate, and mixtures of any two or more of the foregoing. Preferred permanganate salts are sodium permanganate and potassium permanganate; especially preferred is potassium permanganate because of its ready availability and low cost. The permanganate salt(s) can be mixed with a 1-bromopropane product mixture in solid form or as a pre-formed solution of the permanganate salt.

Typically, permanganate is mixed with or contacted with a 1-bromopropane product mixture in the practice of this invention in proportions of 0.1 to 5 parts by weight permanganate per 1000 parts by weight of 1-bromopropane product mixture. Preferably, permanganate is mixed with a 1-bromopropane product mixture in proportions of 0.25 to 3 parts by weight permanganate per 1000 parts by weight of 1-bromopropane product mixture; more preferably, permanganate is mixed with a 1-bromopropane product mixture in proportions of 0.75 to 2.5 parts by weight of permanganate per 1000 parts by weight of 1-bromopropane product mixture. Larger amounts of permanganate can be used in the practice of this invention, but are not particularly desired, as excellent results are achieved with the amounts just described.

In the practice of the invention, when a permanganate salt is mixed with a 1-bromopropane product mixture as a pre-formed solution, normally and preferably an aqueous solution, the permanganate salt concentration in the solution is typically in the range of 0.01 N to 1 N. Preferably, the concentration of the permanganate salt in the solution is 0.025 N to 0.5 N.

Various water-soluble inorganic bases may be used in the aqueous solution of inorganic base. Such inorganic bases include oxides, hydroxides, acetates, sulfates, carbonates, and bicarbonates of the alkali metals, alkaline earth metals, zinc, ammonium, and the like. Examples of suitable bases include sodium oxide, potassium oxide, magnesium oxide, calcium oxide, zinc oxide, ammonium hydroxide, lithium hydroxide, sodium hydroxide, potassium hydroxide, rubidium hydroxide, magnesium hydroxide, calcium hydroxide, barium hydroxide, zinc hydroxide, ammonium nitrate, ammonium sulfate, lithium acetate, lithium carbonate, sodium acetate, sodium bicarbonate, sodium carbonate, potassium acetate, potassium bicarbonate, potassium carbonate, potassium nitrite, potassium sulfite, rubidium carbonate, cesium acetate, cesium bicarbonate, magnesium carbonate, calcium carbonate, and zinc carbonate. Mixtures of two or more inorganic bases can be used. Alkali metal bases are preferred; more preferred are alkali metal hydroxides, and sodium hydroxide and potassium hydroxide are highly preferred as inorganic bases. Typically, the aqueous solution of inorganic base has in the range of 15 to 75 weight percent inorganic base, and the aqueous solution of inorganic base preferably has in the range of 25 to 55 weight percent inorganic base.

The term 1,2-epoxide does not mean that the ring must involve the carbon atoms in the 1- and 2-positions; instead this means that the epoxide (cyclic ether) has three atoms in the ring rather than 4 atoms in the ring. Examples of suitable 1,2-epoxides include alkylene oxides and/or cycloalkylene oxides of up to 8 carbon atoms, including propylene oxide, butylene oxide, pentene oxide, hexene oxide, heptene oxide, octene oxide, cyclopentene oxide, cyclohexene oxide, methyl-1,2-cyclopeatene oxide or mixtures thereof. Preferably, the 1,2-epoxide is butylene oxide, irrespective of whether the butylene oxide is 1,2-epoxybutane or 2,3-epoxybutane or a mixture of both.

Typically, enough 1,2-epoxide is mixed with the 1-bromopropane product to make a concentration of 1,2-epoxide in the 1-bromopropane product in the range of 10 to 1000 ppm (wt/wt), and preferably in the range of 100 to 600 ppm (wt/wt). More preferably, the 1,2-epoxide concentration is in the range of 250 to 500 ppm (wt/wt); still more preferred is a 1,2-epoxide concentration in the 1-bromopropane product in the range of 400 to 500 ppm (wt/wt).

The use of a 1,2-epoxide is optional in the practice of this invention. Especially for long periods of storage and/or exposure to high temperature (*e.g*., outdoor summer temperatures), the presence of a 1,2-epoxide in a 1-bromopropane product is desirable, but absent such conditions, the permanganate treatment alone may be sufficient to keep the acidity of a 1-bromopropane product within desirable limits.

Preferred processes of the invention include those in which a 1,2-epoxide is mixed with a 1-bromopropane product in an amount to make a concentration of 250 ppm to 500 ppm, especially where the 1,2-epoxide is butylene oxide; preferably, permanganate is sodium permanganate or potassium permanganate. More preferably, the processes further comprise drying the 1-bromopropane product until the water content of the 1-bromopropane product is less than about 100 ppm.

Other preferred processes of the invention comprise those in which permanganate is mixed with the 1-bromopropane product mixture in proportions of 0.1 to 5 parts by weight permanganate per 1000 parts by weight of 1-bromopropane product mixture, and wherein a 1,2-epoxide is mixed with a 1-bromopropane product in an amount to make a concentration of 250 ppm to 500 ppm, where the 1,2-epoxide is butylene oxide; preferably, the permanganate is sodium permanganate or potassium permanganate. More preferably, these processes further comprise drying the 1-bromopropane product until the water content of the 1-bromopropane product is less than about 100 ppm.

Still other preferred processes of the invention comprise those in which permanganate is mixed with a 1-bromopropane product mixture in proportions of 0.1 to 5 parts by weight permanganate per 1000 parts by weight of 1-bromopropane product mixture, wherein the permanganate is sodium permanganate or potassium permanganate, and wherein a 1,2-epoxide is mixed with the 1-bromopropane product in an amount to make a concentration of 250 ppm to 500 ppm. More preferably, the processes further comprise drying the 1-bromopropane product until the water content of the 1-bromopropane product is less than about 100 ppm.

In especially preferred processes of this invention, only water, one or more aqueous solutions of at least one inorganic base, and permanganate are brought into contact with a 1-bromopropane product mixture. It is preferred that only water and one or more aqueous solutions of at least one inorganic base are brought into contact with the 1-bromopropane product.

During the mixing of permanganate with a 1-bromopropane product mixture, pursuant to this invention, a new composition of matter is formed, comprising permanganate and the 1-bromopropane product mixture; this composition is a permanganate-containing 1-bromopropane product mixture. It is to be understood that the composition encompasses permanganate in whatever form into which the permanganate may be transformed when combined with a 1-bromopropane product mixture, and the 1-bromopropane product mixture.

Other preferred compositions of the invention comprising permanganate and a 1-bromopropane product mixture include those in which permanganate is present in proportions of 0.1 to 5 parts by weight per 1000 parts by weight of the 1-bromopropane product mixture. Still more preferred compositions of the invention comprising permanganate and a 1-bromopropane product mixture are those in which permanganate is present in proportions of 0.25 to 3 parts by weight per 1000 parts by weight of 1-bromopropane product mixture. In especially preferred compositions of the invention comprising permanganate and a 1-bromopropane product mixture, only permanganate and the 1-bromopropane product mixture are present in the composition.

After the mixing of permanganate with a 1-bromopropane product mixture and the subsequent removal of the permanganate in the processes of this invention, a 1-bromopropane product that has been contacted with permanganate is produced.

The following examples are presented for purposes of illustration.

The 60 ° C stability test (also sometimes called the acidity test) used in the following Examples was conducted as follows: A quantity of about 160 grams of the finished 1-bromopropane product to be tested was placed in a 4 fluid ounce (118 mL) Boston Round screw cap bottle. The Teflon^{®} polymer-lined cap for the bottle was applied without excluding air from the free head space. The capped bottle was held in a 60°C oven for 30 days, opening it once at 10 days to take a sample for analysis. The sample was then allowed to cool to room temperature before determining the acidity. The analysis for acidity involved shaking 80-120 grams of the cooled test sample with 30 to 50 mL of ultra pure water followed by phase separation and titration of the aqueous phase with 0.01 N NaOH to the phenolphthalein endpoint. This acidity analysis will show a positive result for HBr, as well as for carboxylic acids such as propionic acid.

The gas chromatography analyses of the 1-bromopropane products in the following Examples were performed on a Hewlett-Packard 5890 gas chromatograph equipped with a split injector, flame ionization detector and a 30M x 0.53 mm x 3µm DB-624 capillary column operating at 35°C. The temperature was held at 35°C for 8 minutes, then raised at 10°C/min. to 230 ° C, final time 3 minutes. The column head pressure was 4.5 psig (1.32x10⁵ Pa) and the total flow of He was 75 mL/minute. An injection volume of 0.5 µL of neat sample was used. The amounts of 2-bromopropane, propanol, propionaldehyde, and propionic acid reported in the Examples below were determined by gas chromatography (GC), and are based on GC area percent, unless otherwise stated.

In the following Examples, 1-bromopropane products were analyzed for the presence of KMnO₄ using inductively coupled plasma (ICP) analysis for manganese, and the water content of the 1-bromopropane products were determined by coulometric Karl Fisher analysis.

Comparative Example A illustrates a typical process for preparing 1-bromopropane products from propanol and HBr, and Table A shows the amounts of impurities in a 1-bromopropane product at the end of a process and after storage when the 1-bromopropane product mixture is not mixed with permanganate. Examples 1-3 show processes of the invention.

### COMPARATIVE EXAMPLE A

A 1-bromopropane product was prepared from 1-propanol and HBr as follows:
(1) 1-Propanol (9000 L) was charged to a reactor. A maximum HBr flow (200 m³.h⁻¹) to the reactor was started while the temperature of the cooled and agitated mixture in the reactor was kept at < 45°C. After the reaction was complete, the mixture was cooled to 30°C. Water (300 L) was added and the mixture was agitated. Agitation was stopped, and the mixture was allowed to settle. The resultant organic and aqueous phases were separated; the aqueous phase was discarded.
(2) While agitating the organic phase (the 1-bromopropane product mixture), aqueous NaOH (48%, 500 L) and water (100 L) were added to the organic phase (1-bromopropane product mixture). Agitation was stopped, the layers were allowed to separate, and the aqueous layer was discarded.
(3) Distillation of the organic layer (12500 kg) was started; the first 300 liters collected were discarded. The distilled organic layer was made to flow into water (1000 kg) already being stirred, until the temperature in the distillation column head reached 80°C. Aqueous NaOH (23%) was added to the mixture of distillate (12000 kg) and water (1000 kg) until the pH was between 8 and 10. The mixture was agitated. Once agitation was stopped, the mixture was allowed to settle. The resultant organic and aqueous phases were separated; the aqueous phase was discarded.
(4) Water was added to the organic phase, and the mixture was agitated. Aqueous NaOH (23 wt%) was added to the mixture until the pH was between 8 and 10. The mixture was agitated. Once agitation was stopped, the mixture was allowed to settle. The resultant organic and aqueous phases were separated; the aqueous phase was discarded.
(5) The organic phase was dried by passing it through a column containing CaCl₂ until the final water content of the 1-bromopropane product was less than 80 ppm. Enough butylene oxide was added to the 1-bromopropane product mixture to make a butylene oxide concentration of about 450-500 ppm.

No permanganate was added during or after the preparation. Results for the 1-bromopropane product initially as well as after 10 days and after 30 days at 60°C are summarized in Table A, as amounts present in the 1-bromopropane product.

**TABLE A**

| | Amount present at start of stability test | Amount present after 10 days at 60°C | Amount present after 30 days at 60°C |
|---|---|---|---|
| 1-Bromopropane | 99.88% | 99.87% | 99.87% |
| 2-Bromopropane | 0.0161% | 0.0157% | 0.0159% |
| Propanol | 0.0175% | 0.0168% | 0.0163% |
| Acidity* | 5 ppm | 40 ppm | 60 ppm |
| Propionaldehyde | 11 ppm | 7 ppm | 10 ppm |
| Propionic acid | 0 ppm | 11 ppm | 25 ppm |

| | | | |
|---|---|---|---|
| *Acidity is reported as ppm HBr. | | | |

A desirable variation on the process here described involves the addition of half of the desired amount of 1,2-epoxide to the 1-bromopropane product mixture prior to the distillation and the addition of the other half of the desired amount of 1,2-epoxide after the distillation of the 1-bromopropane product mixture.

### EXAMPLE 1

Step (1) of Comparative Example A was carried out to form a 1-bromopropane product mixture. Then an aqueous alkaline solution of KMnO₄ (421 g; 0.05 N) was added with stirring to the 1-bromopropane product mixture (2700 g). As a result of this operation, the amount of propanol present in the 1-bromopropane product mixture decreased from 100 ppm to 47 ppm, and no propionaldehyde remained (from an initial amount of propionaldehyde of 7 ppm) in the 1-bromopropane product mixture. The 1-bromopropane product mixture was then washed with aqueous caustic (120 g; 40 wt% NaOH), after which no more propionic acid was present in the 1-bromopropane product mixture. Distillation of the organic layer (2628 g) was started; the first 143 g collected were discarded. Distillation was continued until the temperature in the distillation column head reached 80 °C. Aqueous NaOH (32 mL; 23 wt%) was added to the distillate (2233 g). This mixture was agitated for 1 hour. Once agitation was stopped, the mixture was allowed to settle. The resultant organic and aqueous phases were separated; the aqueous phase was discarded. Water (200 mL) was added to the organic phase, and the mixture was agitated. Aqueous NaOH (23 wt%) was added to the mixture until the pH was between 8 and 10. The mixture was agitated. Once agitation was stopped, the mixture was allowed to settle. The resultant organic and aqueous phases were separated; the aqueous phase was discarded. The organic phase was dried by passing it through a column containing CaCl₂ until the final water content was less than 80 ppm. Enough butylene oxide was added to the 1-bromopropane product mixture to make a butylene oxide concentration of about 450-500 ppm, after which the obtained 1-bromopropane product was subj ected to the acidity test described above. Results for the 1-bromopropane product initially as well as after 10 days and after 30 days at 60°C are summarized in Table 1, as amounts present in the 1-bromopropane product.

**TABLE 1**

| | Amount present at start of stability test | Amount present after 10 days at 60 ° C | Amount present after 30 days at 60 ° C |
|---|---|---|---|
| 1-Bromopropane | 99.9% | 99.9% | 99.9% |
| 2-Bromopropane | 181 ppm | 182 ppm | 183 ppm |
| Propanol | 23 ppm | 22 ppm | 23 ppm |
| Acidity | 1.8 ppm | 9.1 ppm | 3 ppm |
| Propionaldehyde | 0 ppm | 0 ppm | 0 ppm |
| Propionic acid | 0 ppm | 0 ppm | 0 ppm |

| | | | |
|---|---|---|---|
| *Acidity is reported as ppm HBr. | | | |

It can be seen from Table 1 that propionaldehyde and propionic acid are not formed in detectable amounts during the stability test in a 1-bromopropane product obtained from a permanganate-treated 1-bromopropane product mixture.

### EXAMPLE 2

A 1-bromopropane product mixture was prepared as described above in Comparative Example A. After step (3), the 1-bromopropane product mixture was washed with an aqueous alkaline solution of KMnO₄. 168 Grams of an aqueous alkaline solution of KMnO₄ (0.05 N) were used per 1150 g of 1-bromopropane product mixture. After the KMnO₄ wash, when the aqueous and organic phases separated, the upper layer was the aqueous phase. Steps (4) and (5) of the preparation were then carried out, including the addition of the butylene oxide. Results for the 1-brornopropane product initially as well as after 10 days and after 3 0 days at 60 °C are summarized in Table 2, as amounts present in the 1-bromopropane product.

**TABLE 2**

| | Amount present at start of stability test | Amount present after 10 days at 60 °C | Amount present after 30 days at 60°C |
|---|---|---|---|
| 1-Bromopropane | 99.87% | 99.83% | 99.84% |
| 2-Bromopropane | 0.0139% | 0.0139% | 0.0140% |
| Propanol | 0 ppm | 0 ppm | 0 ppm |
| Acidity* | 2.3 ppm | 3.0 ppm | 3.2 ppm |
| Propionaldehyde | 0 ppm | 0 ppm | 0 ppm |
| Propionic acid | 0 ppm | 0 ppm | 0 ppm |

| | | | |
|---|---|---|---|
| *Acidity is reported as ppm HBr. | | | |

### EXAMPLE 3

Three runs were performed, each with a different amount of KMnO₄. A 1-bromopropane product mixture was prepared according to preparation in Comparative Example A. The KMnO₄ was added after step (3). To mix the 1-bromopropane product mixture with the solid KMnO₄, the 1-bromopropane product mixture (1150 g) was added to water (88.5 g). Aqueous NaOH (23 wt%) was added until the pH was between 8 and 10. Once this pH was obtained, solid KMnO₄ was added, and the mixture was stirred during 3 hours. The organic phase was extracted. Referring again to the preparation above, step (4) was omitted; step (5) was carried out, including the addition of butylene oxide. Results for the 1-bromopropane products initially as well as after 10 days and after 30 days at 60 °C are summarized in Table 3, as amounts present in the 1-bromopropane product.

While the acidity in Runs 1 and 2 does increase above 10 ppm by the 30th day at 60 °C, the acidity in these two Runs is much less than that observed in 1-bromopropane product mixtures that have not been treated with permanganate (see Comparative Example A, above).

**TABLE 3**

| Run 1 (0.24 g KMnO₄ per kg 1-bromopropane) | Amount present at start of stability test | Amount present after 10 days at 60 °C | Amount present after 30 days at 60 °C |
|---|---|---|---|
| 1-Bromopropane | 99.88% | 99.87% | 99.87% |
| 2-Bromopropane | 0.0138% | 0.0139% | 0.0140% |
| Propanol | 0 ppm | 0 ppm | 0 ppm |
| Acidity* | 4 ppm | 11.5 ppm | 18.6 ppm |
| Propionaldehyde | 0 ppm | 0 ppm | 3 ppm |
| Propionic acid | 0 ppm | 0 ppm | 0 ppm |

| Run 2 (0.8 g KMnO₄ per kg 1-bromopropane) | | | |
|---|---|---|---|
| 1-Bromopropane | 99.91% | 99.85% | 99.87% |
| 2-Bromopropane | 0.0121% | 0.0126% | 0.0121% |
| Propanol | 0 ppm | 0 ppm | 0 ppm |
| Acidity* | 4 ppm | 21 ppm | 30.2 ppm |
| Propionaldehyde | 0 ppm | 2 ppm | 3 ppm |
| Propionic acid | 0 ppm | 0 ppm | 0 ppm |

| Run 3 (1.6 g KMnO₄ per kg 1-bromopropane) | | | |
|---|---|---|---|
| 1-Bromopropane | 99.92% | 99.90% | 99.90% |
| 2-Bromopropane | 0.014% | 0.0139% | 0.0140% |
| Propanol | 0 ppm | 0 ppm | 0 ppm |
| Acidity* | 3 ppm | 4.4 ppm | 3 ppm |
| Propionaldehyde | 0 ppm | 0 ppm | 0 ppm |
| Propionic acid | 0 ppm | 0 ppm | 0 ppm |

| | | | |
|---|---|---|---|
| *Acidity is reported as ppm HBr. | | | |

## Claims

1. A process for purifying a 1-bromopropane product mixture resulting from a 1-bromopropane production process from 1-propanol and hydrogen bromide, **characterized by** mixing permanganate with said 1-bromopropane product mixture to form a permanganate-containing 1-bromopropane product mixture, and recovering a 1-bromopropane product from said permanganate-containing 1-bromopropane product mixture, wherein said permanganate is mixed with said 1-bromopropane product mixture in proportions of 0.1 to 5 parts by weight permanganate per 1000 parts by weight of 1-bromopropane product mixture.

2. A process as in Claim 1 wherein said mixing is under basic conditions.

3. A process as in Claim 2 wherein said basic conditions involve a pH in the range of 8 to 10.

4. A process as in Claim 1 wherein the permanganate is in solid form.

5. A process as in Claim 1 wherein said permanganate is sodium permanganate or potassium permanganate.

6. A process as in Claim 1 wherein the permanganate is in solid form, and wherein said permanganate is sodium permanganate or potassium permanganate.

7. A process as in Claim 1 wherein said permanganate is sodium permanganate or potassium permanganate, and wherein said permanganate is mixed with said 1-bromopropane product mixture in proportions of 0.1 to 5 parts by weight permanganate per 1000 parts by weight of 1-bromopropane product mixture.

8. A process as in Claim 1 wherein the 1-bromopropane product mixture is washed at least once with water or with an aqueous solution of at least one inorganic base prior to said mixing.

9. A process as in Claim 1 wherein the 1-bromopropane product is washed at least once with water or with an aqueous solution of at least one inorganic base.

10. A process as in Claim 1 wherein the 1-bromopropane product mixture is washed at least once with water or with an aqueous solution of at least one inorganic base prior to said mixing, and wherein the 1-bromopropane product is washed at least once with water or with an aqueous solution of at least one inorganic base.

11. A process as in Claim 8, 9, or 10 wherein said wash is with an aqueous solution of at least one inorganic base, and wherein said inorganic base is an alkali metal base.

12. A process as in Claim 8, 9, or 10 wherein said inorganic base is an alkali metal hydroxide.

13. A process as in Claim 12 wherein said alkali metal hydroxide is sodium hydroxide or potassium hydroxide.

14. A process as in Claim 1 wherein only water, one or more aqueous solutions of at least one inorganic base, and permanganate are brought into contact with the 1-bromopropane product mixture.

15. A process as in Claim 1 wherein only water and one or more aqueous solutions of at least one inorganic base are brought into contact with the 1-bromopropane product.

16. A process as in Claim 1 wherein said permanganate is sodium permanganate or potassium permanganate, wherein said permanganate is mixed with said 1-bromopropane product mixture in proportions of 0.1 to 5 parts by weight permanganate per 1000 parts by weight of 1-bromopropane product mixture, and wherein only water, one or more aqueous solutions of at least one inorganic base, and permanganate are brought into contact with the 1-bromopropane product mixture.

17. A process as in Claim 1 wherein said mixing is carried out by adding permanganate to said 1-bromopropane product mixture.

18. A process as in Claim 1 wherein said permanganate is sodium permanganate or potassium permanganate, wherein said permanganate is mixed with said 1-bromopropane product mixture in proportions of 0.1 to 5 parts by weight permanganate per 1000 parts by weight of 1-bromopropane product mixture, and wherein said mixing is carried out by adding permanganate to said 1-bromopropane product mixture.

19. A process as in Claim 1 wherein said process further comprises drying the 1-bromopropane product until it has a water content of less than about 100 ppm (wt/wt).

20. A process as in Claim 1 wherein said permanganate is sodium permanganate or potassium permanganate, wherein said permanganate is mixed with said 1-bromopropane product mixture in proportions of 0.1 to 5 parts by weight permanganate per 1000 parts by weight of 1-bromopropane product mixture, and wherein said process further comprises drying the 1-bromopropane product until it has a water content of less than about 100 ppm (wt/wt).

21. A process as in Claim 1 wherein at least one 1,2-epoxide is mixed with the 1-bromopropane product.

22. A process as in Claim 21 wherein said 1,2-epoxide is butylene oxide.

23. A process as in Claim 21 wherein said 1,2-epoxide is mixed in an amount to make a concentration of 1,2-epoxide in the 1-bromopropane product in the range of 100 ppm to 600 ppm (wt/wt).

24. A process as in Claim 22 wherein said 1,2-epoxide is mixed in an amount to make a concentration of 1,2-epoxide in the 1-bromopropane product in the range of 250 ppm to 500 ppm (wt/wt).

25. A process as in Claim 22 wherein only water, one or more aqueous solutions of at least one inorganic base, and permanganate are brought into contact with the 1-bromopropane product mixture.

26. A process as in Claim 1 wherein said permanganate is sodium permanganate or potassium permanganate, wherein said mixing is under basic conditions, and wherein said basic conditions involve a pH in the range of 8 to 10.

27. A process as in Claim 26 wherein the 1-bromopropane product mixture is washed at least once with water or with an aqueous solution of at least one inorganic base prior to said mixing, and wherein the 1-bromopropane product is washed at least once with water or with an aqueous solution of at least one inorganic base.

28. A process as in Claim 1 wherein said permanganate is sodium permanganate or potassium permanganate, wherein said permanganate is mixed with said 1-bromopropane product mixture in proportions of 0.1 to 5 parts by weight permanganate per 1000 parts by weight of 1-bromopropane product mixture, wherein said mixing is under basic conditions, and wherein said basic conditions involve a pH in the range of 8 to 10.

29. A process as in Claim 28 wherein the 1-bromopropane product mixture is washed at least once with water or with an aqueous solution of at least one inorganic base prior to said mixing, and wherein the 1-bromopropane product is washed at least once with water or with an aqueous solution of at least one inorganic base.

30. A process as in Claim 27 or 29 wherein said wash is with an aqueous solution of at least one inorganic base, and wherein said inorganic base is an alkali metal hydroxide.

31. A process as in Claim 30 wherein said alkali metal hydroxide is sodium hydroxide or potassium hydroxide.

32. A process as in Claim 1 further comprising subjecting the 1-bromopropane product to further purification, wherein, after said further purification the 1-bromopropane product has an acidity of less than about 10 ppm as ppm HBr after 30 days at 60°C.

33. A composition formed as in Claim 1 comprising permanganate and a 1-bromopropane product mixture.

34. A composition as in Claim 33 wherein permanganate is present in proportions of 0.1 to 5 parts by weight permanganate per 1000 parts by weight of 1-bromopropane product mixture.

35. A composition as in Claim 33 wherein permanganate is present in proportions of 0.25 to 3 parts by weight permanganate per 1000 parts by weight of 1-bromopropane product mixture.

36. A composition as in any of Claims 33 to 35 wherein only the 1-bromopropane product mixture and permanganate are present in the composition.

## Patentansprüche

1. Verfahren zur Reinigung einer 1-Brompropan-Produktmischung, die durch einen 1-Brompropan-Produktionsprozess aus 1-Propanol und Bromwasserstoff entsteht, **dadurch gekennzeichnet, dass** Permanganat mit der 1-Brompropan-Produktmischung gemischt wird, um permanganathaltige 1-Brompropan-Produktmischung zu bilden, und 1-Brompropan-Produkt aus der permanganathaltigen 1-Brompropan-Produktmischung gewonnen wird, wobei das Permanganat mit der 1-Brompropan-Produktmischung in Verhältnissen von 0,1 bis 5 Gewichtsteile Permanganat je 1000 Gewichtsteile der 1-Brompropan-Produktmischung gemischt wird.

2. Verfahren nach Anspruch 1, bei dem das Mischen unter basischen Bedingungen erfolgt.

3. Verfahren nach Anspruch 2, bei dem die basischen Bedingungen einen pH-Wert im Bereich von 8 bis 10 beinhalten.

4. Verfahren nach Anspruch 1, bei dem das Permanganat in fester Form vorliegt.

5. Verfahren nach Anspruch 1, bei dem das Permanganat Natriumpermanganat oder Kaliumpermanganat ist.

6. Verfahren nach Anspruch 1, bei dem das Permanganat in fester Form vorliegt und bei dem das Permanganat Natriumpermanganat oder Kaliumpermanganat ist.

7. Verfahren nach Anspruch 1, wobei das Permanganat Natriumpermanganat oder Kaliumpermanganat ist und bei dem das Permanganat mit der 1-Brompropan-Produktmischung in Verhältnissen von 0,1 bis 5 Gewichtsteile Permanganat je 1000 Gewichtsteile der 1-Brompropan-Produktmischung gemischt wird.

8. Verfahren nach Anspruch 1, bei dem die 1-Brompropan-Produktmischung mindestens einmal mit Wasser oder mit einer wässrigen Lösung von mindestens einer anorganischen Base vor dem Mischen gewaschen wird.

9. Verfahren nach Anspruch 1, bei dem das 1-Brompropan-Produkt mindestens einmal mit Wasser oder mit einer wässrigen Lösung von mindestens einer anorganischen Base gewaschen wird.

10. Verfahren nach Anspruch 1, bei dem die 1-Brompropan-Produktmischung mindestens einmal mit Wasser oder mit einer wässrigen Lösung von mindestens einer anorganischen Base vor dem Mischen gewaschen wird und bei dem das 1-Brompropan-Produkt mindestens einmal mit Wasser oder mit einer wässrigen Lösung von mindestens einer anorganischen Base gewaschen wird.

11. Verfahren nach Anspruch 8, 9 oder 10, bei dem das Waschen mit einer wässrigen Lösung von mindestens einer anorganischen Base geschieht und bei dem die anorganische Base eine Alkalimetallbase ist.

12. Verfahren nach Anspruch 8, 9 oder 10, bei dem die anorganische Base ein Alkalimetallhydroxid ist.

13. Verfahren nach Anspruch 12, wobei das Alkalimetallhydroxid Natriumhydroxid oder Kaliumhydroxid ist.

14. Verfahren nach Anspruch 1, bei dem nur Wasser, eine oder mehrere wässrige Lösungen von mindestens einer anorganischen Base, und Permanganat mit der 1-Brompropan-Produktmischung in Kontakt gebracht werden.

15. Verfahren nach Anspruch 1, bei dem nur Wasser und eine oder mehrere wässrige Lösungen von mindestens einer anorganischen Base mit dem 1-Brompropan-Produkt in Kontakt gebracht werden.

16. Verfahren nach Anspruch 1, bei dem das Permanganat Natriumpermanganat oder Kaliumpermanganat ist, bei dem das Permanganat mit der 1-Brompropan-Produktmischung in Verhältnissen von 0,1 bis 5 Gewichtsteilen Permanganat je 1000 Gewichtsteilen der 1-Brompropan-Produktmischung gemischt wird und bei dem nur Wasser, eine oder mehrere wässrige Lösungen von mindestens einer anorganischen Base, und Permanganat mit der 1-Brompropan-Produktmischung in Kontakt gebracht werden.

17. Verfahren nach Anspruch 1, bei dem das Mischen dadurch ausgeführt wird, dass Permanganat zu der 1-Brompropan-Produktmischung gegeben wird.

18. Verfahren nach Anspruch 1, bei dem das Permanganat Natriumpermanganat oder Kaliumpermanganat ist, bei dem das Permanganat mit der 1-Brompropan-Produktmischung in Verhältnissen von 0,1 bis 5 Gewichtsteilen Permanganat je 1000 Gewichtsteilen der 1-Brompropan-Produktmischung gemischt wird und bei dem das Mischen dadurch ausgeführt wird, dass Permanganat zu der 1-Brompropan-Produktmischung gegeben wird.

19. Verfahren nach Anspruch 1, bei dem das Verfahren ferner Trocknen des 1-Brompropan-Produktesumfasst, bis es einen Wassergehalt von weniger als 100 ppm (Gew./Gew.) aufweist.

20. Verfahren nach Anspruch 1, bei dem das Permanganat Natriumpermanganat oder Kaliumpermanganat ist, bei dem das Permanganat mit der 1-Brompropan-Produktmischung in Verhältnissen von 0,1 bis 5 Gewichtsteilen Permanganat je 1000 Gewichtsteilen der 1-Brompropan-Produktmischung gemischt wird und bei dem das Verfahren ferner Trocknen des 1-Brompropan-Produktes umfasst, bis es einen Wassergehalt von weniger als 100 ppm (Gew./Gew.) aufweist.

21. Verfahren nach Anspruch 1, bei dem mindestens ein 1,2-Epoxid mit dem 1-Brompropan-Produkt gemischt wird.

22. Verfahren nach Anspruch 21, bei dem das 1,2-Epoxid Butylenoxid ist.

23. Verfahren nach Anspruch 21, bei dem das 1,2-Epoxid in einer Menge zugemischt wird, dass sich eine Konzentration des 1,2-Epoxids in dem 1-Bromopropan-Produkt im Bereich von 100 ppm bis 600 ppm (Gew./Gew.) ergibt.

24. Verfahren nach Anspruch 22, bei dem das 1,2-Epoxid in einer Menge zugemischt wird, dass sich eine Konzentration des 1,2-Epoxids in dem 1-Bromopropan-Produkt im Bereich von 250 ppm bis 500 ppm (Gew./Gew.) ergibt.

25. Verfahren nach Anspruch 22, bei dem nur Wasser, eine oder mehrere wässrige Lösungen von mindestens einer anorganischen Base und Permanganat mit der 1-Brompropan-Produktmischung in Kontakt gebracht werden.

26. Verfahren nach Anspruch 1, bei dem das Permanganat Natriumpermanganat oder Kaliumpermanganat ist, bei dem das Mischen unter basischen Bedingungen erfolgt und bei dem die basischen Bedingungen einen pH-Wert im Bereich von 8 bis 10 beinhalten.

27. Verfahren nach Anspruch 26, bei dem die 1-Brompropan-Produktmischung mindestens einmal mit Wasser oder mit einer wässrigen Lösung von mindestens einer anorganischen Base vor dem Mischen gewaschen wird und bei dem das 1-Brompropan-Produkt mindestens einmal mit Wasser oder mit einer wässrigen Lösung von mindestens einer anorganischen Base gewaschen wird.

28. Verfahren nach Anspruch 1, bei dem das Permanganat Natriumpermanganat oder Kaliumpermanganat ist, bei dem das Permanganat mit der 1-Brompropan-Produktmischung in Verhältnissen von 0,1 bis 5 Gewichtsteilen Permanganat je 1000 Gewichtsteilen der 1-Brompropan-Produktmischung gemischt wird, bei dem das Mischen unter basischen Bedingungen erfolgt und bei dem die basischen Bedingungen einen pH-Wert im Bereich von 8 bis 10 beinhalten.

29. Verfahren nach Anspruch 28, bei dem die 1-Brompropan-Produktmischung mindestens einmal mit Wasser oder mit einer wässrigen Lösung von mindestens einer anorganischen Base vor dem Mischen gewaschen wird und bei dem das 1-Brompropan-Produkt mindestens einmal mit Wasser oder mit einer wässrigen Lösung von mindestens einer anorganischen Base gewaschen wird.

30. Verfahren nach Anspruch 27 oder 29, bei dem das Waschen mit einer wässrigen Lösung von mindestens einer anorganischen Base geschieht und bei dem die anorganische Base ein Alkalimetallhydroxid ist.

31. Verfahren nach Anspruch 30, bei dem das Alkalimetallhydroxid Natriumhydroxid oder Kaliumhydroxid ist.

32. Verfahren nach Anspruch 1, bei dem ferner das 1-Brompropan-Produkt weiterer Reinigung unterzogen wird, wobei das 1-Brompropan-Produkt nach der weiteren Reinigung nach 30 Tagen bei 60 °C eine Azidität von weniger als etwa 10 ppm as ppm Bromwasserstoff aufweist.

33. Zusammensetzung, die gemäß Anspruch 1 gebildet worden ist und Permanganat und eine 1-Brompropan-Produktmischung umfasst.

34. Zusammensetzung nach Anspruch 33, bei dem Permanganat in Verhältnissen von 0,1 bis 5 Gewichtsteilen Permanganat je 1000 Gewichtsteilen der 1-Brompropan-Produktmischung vorhanden ist.

35. Zusammensetzung nach Anspruch 33, bei dem Permanganat in Verhältnissen von 0,25 bis 3 Gewichtsteilen Permanganat je 1000 Gewichtsteilen der 1-Brompropan-Produktmischung vorhanden ist.

36. Zusammensetzung nach einem der Ansprüche 33 bis 35, bei dem nur die 1-Brompropan-Produktmischung und Permanganat in der Zusammensetzung vorhanden sind.

## Revendications

1. Procédé de purification d'un mélange de produits de 1-bromopropane résultant d'un procédé de production de 1-bromopropane à partir du 1-propanol et du bromure d'hydrogéne, **caractérisé par** le mélange de permanganate avec ledit mélange de produits de 1-bromo-propane pour former un mélange de produits de 1-bromo-propane contenant du permanganate, et la récupération d'un produit de 1-bromopropane à partir dudit mélange de produits de 1-bromopropane contenant du permanganate, dans lequel ledit permanganate est mélangé avec ledit mélange de produits de 1-bromopropane dans des proportions de 0,1 à 5 parties en poids de permanganate pour 1000 parties en poids de mélange de produits de 1-bromopropane.

2. Procédé selon la revendication 1, dans lequel le mélange est réalisé dans des conditions basiques.

3. Procédé selon la revendication 2, dans lequel lesdites conditions basiques impliquent un pH situé dans la plage allant de 8 à 10.

4. Procédé selon la revendication 1, dans lequel le permanganate est sous forme solide.

5. Procédé selon la revendication 1, dans lequel ledit permanganate est le permanganate de sodium ou le permanganate de potassium.

6. Procédé selon la revendication 1, dans lequel le permanganate est sous forme solide, et dans lequel ledit permanganate est le permanganate de sodium ou le permanganate de potassium.

7. Procédé selon la revendication 1, dans lequel ledit permanganate est le permanganate de sodium ou le permanganate de potassium, et dans lequel ledit permanganate est mélangé avec ledit mélange de produits de 1-bromopropane dans des proportions de 0,1 à 5 parties en poids de permanganate pour 1000 parties en poids de mélange de produits de 1-bromopropane.

8. Procédé selon la revendication 1, dans lequel le mélange de produits de 1-bromopropane est lavé au moins une fois avec de l'eau ou avec une solution aqueuse d'au moins une base inorganique avant ledit mélange.

9. Procédé selon la revendication 1, dans lequel le produit de 1-bromopropane est lavé au moins une fois avec de l'eau ou avec une solution aqueuse d'au moins une base inorganique.

10. Procédé selon la revendication 1, dans lequel le mélange de produits de 1-bromopropane est lavé au moins une fois avec de l'eau ou avec une solution aqueuse d'au moins une base inorganique avant ledit mélange, et dans lequel le produit de 1-bromopropane est lavé au moins une fois avec de l'eau ou avec une solution aqueuse d'au moins une base inorganique.

11. Procédé selon la revendication 8, 9 ou 10, dans lequel ledit lavage est effectué avec une solution aqueuse d'au moins une base organique, et dans lequel ladite base inorganique est une base de métal alcalin.

12. Procédé selon la revendication 8, 9 ou 10, dans lequel ladite base inorganique est un hydroxyde de métal alcalin.

13. Procédé selon la revendication 12, dans lequel ledit hydroxyde de métal alcalin est l'hydroxyde de sodium ou l'hydroxyde de potassium.

14. Procédé selon la revendication 1, dans lequel seulement de l'eau, une ou plusieurs solutions aqueuses d'au moins une base inorganique et du permanganate sont mis en contact avec le mélange de produits de 1-bromopropane.

15. Procédé selon la revendication 1, dans lequel seulement de l'eau et une ou plusieurs solutions aqueuses d'au moins une base inorganique sont mis en contact avec le produit de 1-bromopropane.

16. Procédé selon la revendication 1, dans lequel ledit permanganate est le permanganate de sodium ou le permanganate de potassium, dans lequel ledit permanganate est mélangé avec ledit mélange de produits de 1-bromopropane dans des proportions de 0,1 à 5 parties en poids de permanganate pour 1000 parties en poids de mélange de produits de 1-bromopropane, et dans lequel seulement de l'eau, une ou plusieurs solutions aqueuses d'au moins une base inorganique et du permanganate sont mis en contact avec le mélange de produits de 1-bromopropane.

17. Procédé selon la revendication 1, dans lequel ledit mélange est réalisé par l'ajout de permanganate audit mélange de produits de 1-bromo-propane.

18. Procédé selon la revendication 1, dans lequel ledit permanganate est le permanganate de sodium ou le permanganate de potassium, dans lequel ledit permanganate est mélangé avec ledit mélange de produits de 1-bromopropane dans des proportions de 0,1 à 5 parties en poids de permanganate pour 1000 parties en poids de mélange de produits de 1-bromopropane, et dans lequel ledit mélange est réalisé par l'ajout de permanganate audit mélange de produits de 1-bromo-propane.

19. Procédé selon la revendication 1, dans lequel ledit procédé comprend en outre le séchage du produit de 1-bromopropane jusqu'à ce que sa teneur en eau soit inférieure à environ 100 ppm (poids/poids).

20. Procédé selon la revendication 1, dans lequel ledit permanganate est le permanganate de sodium ou le permanganate de potassium, dans lequel ledit permanganate est mélangé avec ledit mélange de produits de 1-bromopropane dans des proportions de 0,1 à 5 parties en poids de permanganate pour 1000 parties en poids de mélange de produits de 1-bromopropane, et dans lequel ledit procédé comprend en outre le séchage du produit de 1-bromopropane jusqu'à ce que sa teneur en eau soit inférieure à environ 100 ppm (poids/poids).

21. Procédé selon la revendication 1, dans lequel au moins un 1,2-époxyde est mélangé avec le produit de 1-bromopropane.

22. Procédé selon la revendication 21, dans lequel ledit 1,2-époxyde est l'oxyde de butylène.

23. Procédé selon la revendication 21, dans lequel dans lequel ledit 1,2-époxyde est mélangé en une quantité permettant d'obtenir une concentration en 1,2-époxyde dans le produit de 1-bromopropane située dans la plage allant de 100 ppm à 600 ppm (poids/poids).

24. Procédé selon la revendication 22, dans lequel dans lequel ledit 1,2-époxyde est mélangé en une quantité permettant d'obtenir une concentration en 1,2-époxyde dans le produit de 1-bromopropane située dans la plage allant de 250 ppm à 500 ppm (poids/poids).

25. Procédé selon la revendication 22, dans lequel seulement de l'eau, une ou plusieurs solutions aqueuses d'au moins une base inorganique et du permanganate sont mis en contact avec le mélange de produits de 1-bromopropane.

26. Procédé selon la revendication 1, dans lequel ledit permanganate est le permanganate de sodium ou le permanganate de potassium, dans lequel ledit mélange est réalisé dans des conditions basiques, et dans lequel lesdites conditions basiques impliquent un pH situé dans la plage allant de 8 à 10.

27. Procédé selon la revendication 26, dans lequel le mélange de produits de 1-bromopropane est lavé au moins une fois avec de l'eau ou avec une solution aqueuse d'au moins une base inorganique avant ledit mélange, et dans lequel le produit de 1-bromopropane est lavé au moins une fois avec de l'eau ou avec une solution aqueuse d'au moins une base inorganique.

28. Procédé selon la revendication 1, dans lequel ledit permanganate est le permanganate de sodium ou le permanganate de potassium, dans lequel ledit permanganate est mélangé avec ledit mélange de produits de 1-bromopropane dans des proportions de 0,1 à 5 parties en poids de permanganate pour 1000 parties en poids de mélange de produits de 1-bromopropane, dans lequel ledit mélange est réalisé dans des conditions basiques, et dans lequel lesdites conditions basiques impliquent un pH situé dans la plage allant de 8 à 10.

29. Procédé selon la revendication 28, dans lequel le mélange de produits de 1-bromopropane est lavé au moins une fois avec de l'eau ou avec une solution aqueuse d'au moins une base inorganique avant ledit mélange, et dans lequel le produit de 1-bromopropane est lavé au moins une fois avec de l'eau ou avec une solution aqueuse d'au moins une base inorganique.

30. Procédé selon la revendication 27 ou 29, dans lequel ledit lavage est réalisé avec une solution aqueuse d'au moins une base inorganique, et dans lequel ladite base inorganique est un hydroxyde de métal alcalin.

31. Procédé selon la revendication 30, dans lequel ledit hydroxyde de métal alcalin est l'hydroxyde de sodium ou l'hydroxyde de potassium.

32. Procédé selon la revendication 1, comprenant en outre la soumission du produit de 1-bromo-propane à une purification supplémentaire, dans laquelle, après ladite purification supplémentaire, le produit de 1-bromopropane présente une acidité inférieure à environ 10 ppm exprimée en ppm de HBr après 30 jours à 60°C.

33. Composition formée selon la revendication 1, comprenant du permanganate et un mélange de produits de 1-bromopropane.

34. Composition selon la revendication 33, dans laquelle le permanganate est présent dans des proportions de 0,1 à 5 parties en poids de permanganate pour 1000 parties en poids de mélange de produits de 1-bromopropane.

35. Composition selon la revendication 33, dans laquelle le permanganate est présent dans des proportions de 0,25 à 3 parties en poids de permanganate pour 1000 parties en poids de mélange de produits de 1-bromopropane.

36. Composition selon l'une quelconque des revendications 33 à 35, dans laquelle le mélange de produits de 1-bromopropane et le permanganate sont seulement présents dans la composition.
